# EUROPÄISCHE PATENTANMELDUNG

(11) **EP 1 813 252 A1**
(43) Veröffentlichungstag der Anmeldung: **01.08.2007**
(21) Anmeldenummer: 06025542.9
(22) Anmeldetag: 11.12.2006
(51) Int. Cl.: A61K 8/04, A61K 8/81, A61Q 1/12, A61Q 5/00, A61Q 19/00

(54) **Kosmetische Zusammensetzung zur Mattierung der Kopfhaut**

(30) Priorität: 30.01.2006 DE 102006004355
(71) Anmelder: Henkel Kommanditgesellschaft auf Aktien, 40589 Düsseldorf (DE)
(72) Erfinder: Delowsky, Jens, 22844 Norderstedt (DE); Träger, Anemone, 40227 Düsseldorf (DE)

(57) **Zusammenfassung**

Die vorliegende Anmeldung betrifft die Verwendung topischer kosmetischer oder dermatologischer Zusammensetzungen zur verbesserten Behandlung sebumglänzender Kopfhaut (Skalp), insbesondere zur langanhaltenden Mattierung der Kopfhaut, wobei die Zusammensetzungen in einem geeigneten kosmetischen oder dermatologischen Träger mindestens einen partikelförmigen, sebumsorbierenden Wirkstoff enthalten.

## Beschreibung

Die Erfindung betrifft die Verwendung topischer kosmetischer oder dermatologischer Zusammensetzungen zur verbesserten Behandlung sebumglänzender Kopfhaut (Skalp), insbesondere zur langanhaltenden Mattierung der Kopfhaut, wobei die Zusammensetzungen in einem geeigneten kosmetischen oder dermatologischen Träger mindestens einen partikelförmigen, sebumsorbierenden Wirkstoff enthalten.

Bei sogenannter fettiger Haut sondern die Talgdrüsen zuviel Fett ab, das sich als dünner Film auf der Hautoberfläche ausbreitet. Ein Merkmal fettiger Haut ist daher ein übermäßiger Hautglanz. Dieser tritt im Gesicht insbesondere in der sogenannten T-Zone (Stirn, Nase, Kinn) auf. Übermäßiger Hautglanz stellt aber auch ein ästhetisches Problem der unbehaarten oder dünn behaarten Kopfhaut dar. Dies ist insbesondere unerwünscht von Glatzenträgern oder Halbglatzenträgern oder Menschen mit deutlich ausgedünntem Haarwuchs oder Kahlstellen auf der Kopfhaut, da eine glänzende Kopfhaut den - häufig als (optisch) unangenehm empfundenen - Mangel an Haarwuchs optisch hervorhebt.

Verfahren zur Mattierung der Kopfhaut sind im Stand der Technik bislang noch nicht bekannt.

Die Talgproduktion der Talgdrüsen wird hormonell gesteuert. Die Hauptursache für fettige Haut sind häufig die Geschlechtshormone, und zwar sowohl die weiblichen (Östrogene) als auch die männlichen (Androgene).

Eine gewisse Menge an Sebum ist physiologisch notwendig, um die Haut nicht austrocknen zu lassen und gehört auch zu einem gesunden Hauterscheinungsbild. Eine Sebum-freie Haut sieht trocken aus und ist häufig mit einem unangenehmen Spannungsgefühl und Juckreiz verbunden. Zuviel Sebum ist jedoch ebenfalls unerwünscht, insbesondere aus ästhetischen Gründen, aber auch, weil es einen Nährboden für Hautunreinheiten, Pickel, Mitesser und Aknebakterien bietet.

Es besteht ein großes Interesse an wirksamen kosmetischen Produkten, die in der Lage sind, das Erscheinungsbild fettiger, sebumglänzender Kopfhaut schnell wirkend und langanhaltend zu verbessern, insbesondere zu mattieren.

Unter "Kopfhaut" wird erfindungsgemäß die Haut der Kopfschwarte beziehungsweise des Skalps verstanden, die das Schädeldach (Calvaria) bedeckt.

Eine Aufgabe der vorliegenden Erfindung war es, das Erscheinungsbild von wenig behaarter oder von Haarausfall betroffener oder rasierter Kopfhaut zu verbessern.
Eine weitere Aufgabe der vorliegenden Erfindung war es, topische kosmetische oder dermatologische Zusammensetzungen zur Behandlung sebumglänzender Kopfhaut, insbesondere zur langanhaltenden Kopfhautmattierung, bereitzustellen.

Überraschend und für den Fachmann nicht vorhersehbar wurde nun festgestellt, dass das optische Erscheinungsbild der von Glatzenbildung oder Kahlstellen betroffenen Kopfhaut deutlich verbessert werden kann durch Applikation von Zusammensetzungen zur topischen Behandlung der Haut, die in einem geeigneten kosmetischen oder dermatologischen Träger mindestens einen partikelförmigen, sebumsorbierenden Wirkstoff enthalten.

Gegenstand der vorliegenden Erfindung ist die Verwendung einer kosmetischen oder dermatologischen Zusammensetzung, enthaltend in einem geeigneten kosmetischen oder dermatologischen Träger mindestens einen partikelförmigen, sebumsorbierenden Wirkstoff zur nicht-therapeutischen, kosmetischen topischen mattierenden Behandlung und/oder zur Behandlung fettiger und/oder sebumglänzender Haut der Kopfhaut (Skalp).

Erfindungsgemäß bevorzugte kosmetische oder dermatologische Zusammensetzungen sind dadurch gekennzeichnet, dass der partikelförmige, sebumsorbierende Wirkstoff ausgewählt ist aus anorganischen und organischen kosmetischen Adsorbentien mit mittleren Partikeldurchmessern von 0,1 - 100 µm, bevorzugt 0,5 - 50 µm, besonders bevorzugt 5 - 30 µm und außerordentlich bevorzugt 10 - 25 µm. Besonders bevorzugte anorganische Adsorbentien sind ausgewählt aus Kieselsäuren, insbesondere Aerosil^{®}-Typen, Kieselgelen, Siliciumdioxid, Tonen und Schichtsilicaten, insbesondere Bentoniten oder Kaolin, Magnesiumaluminiumsilikaten, insbesondere Talkum, und Bornitrid, sowie Mischungen der genannten Substanzen. Besonders bevorzugte organische Adsorbentien sind ausgewählt aus Stärken und Stärkederivaten, die chemisch und/oder physikalisch modifiziert sein können, insbesondere modifizierten Stärkederivaten vom Typ DRY FLO^{®} der National Starch and Chemical Company, Cellulosepulvern, Lactoglobulinderivaten, insbesondere Natrium-C₈₋₁₆-Isoalkylsuccinyllactoglobulinsulfonat, z. B. als Handelsprodukt Biopol^{®} OE von Brooks Industries erhältlich, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die Polymerpulver in einer besonders bevorzugten Ausführungsform der Erfindung vernetzt sein können, sowie Mischungen der genannten Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugt verwendete Polymerpulver sind vernetzte Polymethacrylate (insbesondere die Handelsprodukte Micropearl^{®} M von SEPPIC oder Plastic Powder A von NIKKOL), vernetzte Polymethylmethacrylate (Micropearl^{®} 305 und Micropearl^{®} 310 von SEPPIC), Styrol-Divinylbenzol-Copolymere (z. B. Plastic Powder FP von NIKKOL), Polyethylen- und Polypropylen-Pulver (z. B. ACCUREL^{®} EP 400 von AKZO) oder auch Siliconpolymere (z. B. Silicone Powder X2-1605 von Dow Corning).
In einer weiteren bevorzugten Ausführungsform der Erfindung weist der partikelförmige, sebumsorbierende Wirkstoff Hohlräume auf. In einer weiteren bevorzugten Ausführungsform der Erfindung weist der partikelförmige, sebumsorbierende Wirkstoff halbkugelförmige Partikel auf. Bevorzugt sind mindestens 50 Gew.-% der Partikel halbkugelförmig, besonders bevorzugt sind mindestens 80 Gew.-% der Partikel halbkugelförmig. In einer weiteren bevorzugten Ausführungsform der Erfindung weist der partikelförmige, sebumsorbierende Wirkstoff halbkugelförmige Partikel mit Hohlräumen auf, bevorzugt sind mindestens 50 Gew.-% der Partikel halbkugelförmig mit Hohlräumen, besonders bevorzugt sind mindestens 80 Gew.-% der Partikel halbkugelförmig mit Hohlräumen.
Besonders bevorzugte partikelförmige, sebumsorbierende Wirkstoffe sind ausgewählt aus vernetzten Polymethylmethacrylaten. Besonders bevorzugte vernetzte Polymethylmethacrylate weisen einen mittleren Partikeldurchmesser von 0,1 - 100 µm, bevorzugt 0,5 - 50 µm, besonders bevorzugt 5 - 30 µm und außerordentlich bevorzugt 10 - 25 µm auf. Außerordentlich bevorzugt ist die Verwendung mindestens eines der Handelsprodukte Micropearl^{®} 305 und Micropearl^{®} 310 von SEPPIC. Außerordentlich bevorzugte partikelförmige, sebumsorbierende Wirkstoffe sind vernetzte Polymethylmethacrylate mit mittleren Partikeldurchmessern von 0,5 - 50 µm, die halbkugelförmige Partikel mit Hohlräumen umfassen, wobei besonders bevorzugt mindestens 50 Gew.-% der Partikel halbkugelförmig mit Hohlräumen sind. Ein entsprechend besonders bevorzugtes Handelsprodukt ist Micropearl^{®} 310 von SEPPIC.

Erfindungsgemäß besonders bevorzugt verwendete Zusammensetzungen sind dadurch gekennzeichnet, dass sie mindestens einen partikelförmigen sebumsorbierenden Wirkstoff in einer Gesamtmenge von 0,1 bis 10 Gew.-%, bevorzugt 0,5 - 1 bis 7 Gew.-%, besonders bevorzugt 2 bis 5 Gew.-%, und außerordentlich bevorzugt 3 bis 4 Gew.-%, jeweils bezogen auf die gesamte Zusammensetzung, enthalten.

Eine erfindungsgemäß besonders bevorzugte Verwendung ist dadurch gekennzeichnet, dass die erfindungsgemäß verwendeten Zusammensetzungen neben dem mindestens einen partikelförmigen, sebumsorbierenden Wirkstoff mindestens einen sebumregulierenden Wirkstoff enthalten.

Unter sebumregulierenden Wirkstoffen werden erfindungsgemäß Wirkstoffe verstanden, die die Sebumproduktion oder den Sebumaustritt aus den Talgdrüsen reduzieren. Die sebumregulierenden Wirkstoffe liegen in den erfindungsgemäßen Zusammensetzungen in gelöster Form vor, im Unterschied zu den partikelförmigen sebumsorbierenden Wirkstoffen.

Zu den sebumregulierenden Wirkstoffen zählen beispielsweise adstringierende Wirkstoffe, die zu einer temporären Verengung der Hautporen führen, beispielsweise Tannine bzw. Gerbstoffe bzw. allgemein Polyphenole, weiterhin Aluminiumsalze, wie sie auch als Antitranspirant-Wirkstoffe Verwendung finden. Diese Wirkstoffe regulieren die Sebummenge an der Kopfhautoberfläche durch Verzögerung des Sebumaustritts. Adstringierende Wirkstoffe können ein unangenehmes Hautgefühl hervorrufen.

Bestimmte sebumregulierende Wirkstoffe können die Sebumproduktion reduzieren, indem sie z. B. das an der Aktivität der Talgdrüsen beteiligte Enzym 5-alpha-Reductase inhibieren. Allerdings ist das Enzym 5-alpha-Reductase auch an einigen Formen des Haarausfalls beteiligt, so dass derartige Wirkstoffe zur Applikation auf eine von Haarausfall betroffene Hautpartie weniger bevorzugt sind. Erfindungsgemäß besonders bevorzugt ist die Verwendung sebumregulierender Wirkstoffe, die nicht als 5-alpha-Reductase-Inhibitor wirken. Besonders bevorzugt sind die erfindungsgemäß verwendeten Zusammensetzungen frei von 5-alpha-Reductase-Inhibitoren. Der sebumregulierende Wirkstoff Zimtbaumextract (z. B. Sepicontrol^{®} A5 von der Firma Seppic) beispielsweise enthält einen 5-alpha-Reductase-Inhibitor und ist daher erfindungsgemäß weniger bevorzugt.

Erfindungsgemäß bevorzugt verwendete kosmetische oder dermatologische Zusammensetzungen sind dadurch gekennzeichnet, dass der sebumregulierende Wirkstoff ausgewählt ist aus Azelainsäure, Azelainsäurederivaten, insbesondere dem Azelainsäurederivat Potassium Azeloyl Diglycinate, das zum Beispiel als Handelsprodukt Azeloglicina von Sinerga erhältlich ist, Sebacinsäure, 10-Hydroxydecansäure, 1,10-Decandiol, Mischungen aus Sebacinsäure, 10-Hydroxydecansäure und 1,10-Decandiol, wie sie zum Beispiel als Handelsprodukt Acnacidol PG von Vincience erhältlich sind, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin(salzen), Gerbsäure(salzen) und/oder Gallotanninen und Naringin, wie sie zum Beispiel als Handelsprodukt BiSCos Glynarin PF (INCI: AQUA (WATER), ALCOHOL, PHENOXYETHANOL, AMMONIUM GLYCYRRHIZATE, TANNIC ACID, NARINGINE) von der Firma Biesterfeld erhältlich sind, weiterhin aus Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques), Hefeproteinhydrolysaten, wie sie z.B. in den Handelsprodukten der Asebiol^{®}-Serie von Laboratoires Sérobiologiques erhältlich sind, insbesondere Asebiol^{®} LS 2539 BT 2 (INCI: Aqua, Hydrolyzed Yeast Protein, Pyridoxine, Niacinamide, Glycerin, Panthenol, Allantoin, Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin), mindestens einer Vitamin B6-Komponente, die ausgewählt ist aus Pyridoxin, Pyridoxinhydrochlorid, Pyridoxin-5'-monophosphat, Pyridoxal, Pyridoxal-5'-monophosphat, Pyridoxamin, Pyridoxaminhydrochlorid und Pyridoxamin-5'-monophosphat sowie Mischungen dieser Komponenten, Biotin und Biotinestern, und PEG-8 Isolauryl Thioether, wie es z. B. in dem Handelsprodukt "Antifettfaktor^{®} COS-218/2-A" von Cosmetochem enthalten ist (INCI: Aqua, Cetyl-PCA, PEG-8 Isolauryl Thioether, PCA, Cetyl Alcohol).
Erfindungsgemäß besonders bevorzugt verwendete Zusammensetzungen sind dadurch gekennzeichnet, dass sie mindestens einen sebumregulierenden Wirkstoff in einer Gesamtmenge von 0,001 bis 5 Gew.-%, bevorzugt 0,01 bis 2 Gew.-%, besonders bevorzugt 0,05 bis 1,5 Gew.-% und außerordentlich bevorzugt 0,1 - 0,5 Gew.-%, jeweils bezogen auf die gesamte Zusammensetzung, enthalten.

Weitere erfindungsgemäß besonders bevorzugt verwendete kosmetische oder dermatologische Zusammensetzungen sind dadurch gekennzeichnet, dass der partikelförmige sebumsorbierende Wirkstoff ausgewählt ist aus vernetzten Polymethylmethacrylaten mit mittleren Partikeldurchmessern von 5 - 50 µm, die halbkugelförmige Partikel mit Hohlräumen umfassen, wobei besonders bevorzugt mindestens 50 Gew.-% der Partikel halbkugelförmig mit Hohlräumen sind, und dass weiterhin mindestens ein Wirkstoff enthalten ist, der ausgewählt ist aus:
- Vitaminen, Provitaminen und Vitaminvorstufen der Gruppen A, B, C, E, H und K und den Estern und Salzen der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- hautberuhigenden und
- feuchtigkeitsspendenden Wirkstoffen sowie
- Mischungen dieser Wirkstoffe.
Für den Fachmann überraschend wurde festgestellt, dass der Zusatz von mindestens einem dieser genannten Wirkstoffe zu einer weiteren Verbesserung des optischen Erscheinungsbildes der Kopfhaut führt.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff sind dadurch gekennzeichnet, dass sie weiterhin mindestens einen kosmetischen Wirkstoff enthalten, der ausgewählt ist aus mindestens einem Vitamin, Provitamin oder einer als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß bevorzugt sind Vitamin A-Säure und deren Ester, Vitamin A-Aldehyd und Vitamin A-Alkohol sowie dessen Ester, wie Retinylpalmitat und Retinylacetat, weiterhin auch Isotretinoin. Die erfindungsgemäßen Zusammensetzungen enthalten die mindestens eine Vitamin A-Komponente bevorzugt in einer Gesamtmenge von 0,05 - 1 Gew.-%, bezogen auf die gesamte Zusammensetzung. Die Vitamin A-Komponente hat überraschenderweise im Zusammenwirken mit den erfindungsgemäß kombinierten partikelförmigen sebumsorbierenden Wirkstoffen einen unerwarteten positiven Effekt auf die Sebumsorption.

Die erfindungsgemäß bevorzugten Komponenten, die zur Vitamin B-Gruppe oder zu dem Vitamin B-Komplex gehören, sind ausgewählt aus
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

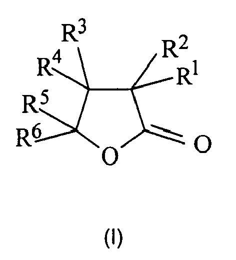

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind.

Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Weitere erfindungsgemäß besonders bevorzugt verwendete Vitamin B6-Komponenten sind ausgewählt aus Pyridoxin, Pyridoxinhydrochlorid, Pyridoxin-5'-monophosphat, Pyridoxal, Pyridoxal-5'-monophosphat, Pyridoxamin, Pyridoxaminhydrochlorid und Pyridoxamin-5'-monophosphat sowie Mischungen dieser Komponenten. In den erfindungsgemäß verwendeten Mitteln ist bevorzugt mindestens eine Vitamin B₆-Komponente in einer Gesamtmenge von 0,0001 bis 1,0 Gew.-%, insbesondere 0,001 bis 0,1 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Die erfindungsgemäß bevorzugten Komponenten, die zur Vitamin C-Gruppe oder zu dem Vitamin C-Komplex gehören, sind ausgewählt aus Vitamin C (Ascorbinsäure) und seinen Derivaten, insbesondere den Estern der Ascorbinsäure mit organischen und anorganischen Säuren und deren Salze, sowie den Acetalen mit Glucose oder anderen Zuckern, insbesondere Ascorbylglucosid. Vitamin C und/oder mindestens eines seiner Derivate wird bevorzugt in einer Gesamtmenge von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Vitamin C-Komponente hat überraschenderweise im Zusammenwirken mit dem erfindungsgemäß verwendeten partikelförmigen, sebumsorbierenden Wirkstoff einen unerwarteten positiven Effekt auf die Sebumsorption. Die Verwendung mindestens eines Mitglieds der Vitamin C - Gruppe in Kombination mit Tocopherolen und/oder anderen Mitgliedern der Vitamin E - Gruppe kann ebenfalls bevorzugt sein.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin mindestens einen kosmetischen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Die genannten Säuren, ihre Ester, Lactone und/oder Salze haben überraschenderweise im Zusammenwirken mit den erfindungsgemäß kombinierten partikelförmigen sebumsorbierenden Wirkstoffen einen unerwarteten positiven Effekt auf die Sebumsorption. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und CalciumSalze.
Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Erfindungsgemäß besonders bevorzugt verwendete kosmetische oder dermatologische Zusammensetzungen zur topischen Behandlung der Kopfhaut, die in einem geeigneten kosmetischen oder dermatologischen Träger mindestens einen sebumsorbierenden Wirkstoff enthalten, sind dadurch gekennzeichnet, dass sie weiterhin mindestens einen kosmetischen Wirkstoff enthalten, der ausgewählt ist aus mindestens einem Flavonoid und/oder mindestens einem Flavonoid-reichen Pflanzenextrakt.
Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)flavon-3-(6-O-(6-deoxy-α-L-mannopyranosyl)-β-D-glucopyranosid)), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin mindestens einen kosmetischen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (II) auf: 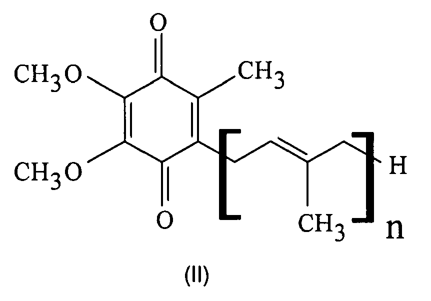 mit n = 6, 7, 8, 9 oder 10.
Ubichinone und/oder Ubichinole haben überraschenderweise im Zusammenwirken mit den erfindungsgemäß kombinierten partikelförmigen sebumsorbierenden Wirkstoffen einen unerwarteten positiven Effekt auf die Sebumsorption. Besonders bevorzugt ist das Ubichinon der Formel (II) mit n = 10, auch bekannt als Coenzym Q10.
Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten. Es wurde überraschend festgestellt, dass der Zusatz von Ectoin zu den erfindungsgemäß verwendeten partikelförmigen sebumsorbierenden Wirkstoffen eine besonders pflegende Wirkung auf die fettige Haut ausübt, so dass bevorzugt auf den Zusatz pflegender kosmetischer Öle und Fette in den erfindungsgemäßen Zusammensetzungen verzichtet werden kann.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin mindestens einen hautberuhigenden Wirkstoff enthalten. Erfindungsgemäß bevorzugt verwendete hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Es wurde überraschend festgestellt, dass der Zusatz von hautberuhigenden Wirkstoffen zu den erfindungsgemäß verwendeten partikelförmigen sebumsorbierenden Wirkstoffen eine besonders pflegende Wirkung auf die fettige Haut ausübt.
Die hautberuhigenden Wirkstoffe sind bevorzugt in Gesamtmengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte erfindungsgemäß verwendete Zusammensetzung, enthalten.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin mindestens einen feuchtigkeitsspendenden Wirkstoff enthalten. Erfindungsgemäß bevorzugte feuchtigkeitsspendende Wirkstoffe sind ausgewählt aus Desoxyzuckern, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, N,N-Bis(2-Hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat.
Es wurde überraschend festgestellt, dass der Zusatz von feuchtigkeitsspendenden Wirkstoffen zu dem erfindungsgemäß verwendeten partikelförmigen sebumsorbierenden Wirkstoff eine besonders pflegende Wirkung auf die fettige Haut ausübt, so dass gegebenenfalls und bevorzugt auf den Zusatz pflegender kosmetischer Öle und Fette in den erfindungsgemäß verwendeten Zusammensetzungen verzichtet werden kann.
Die feuchtigkeitsspendenden Wirkstoffe sind bevorzugt in Gesamtmengen von 0,001 bis 10 Gew.-%, besonders bevorzugt 0,01 bis 5 Gew.-% und außerordentlich bevorzugt 0,1 bis 1 oder 2 Gew.-%, jeweils bezogen auf die gesamte erfindungsgemäß verwendete Zusammensetzung, enthalten.

Ein weiterer Gegenstand der vorliegenden Erfindung ist die Verwendung einer kosmetischen oder dermatologischen Zusammensetzung, die in einem geeigneten kosmetischen oder dermatologischen Träger mindestens einen partikelförmigen, sebumsorbierenden Wirkstoff zur nicht-therapeutischen, kosmetischen topischen mattierenden Behandlung der Kopfhaut/des Skalps und/oder zur nicht-therapeutischen, kosmetischen topischen Behandlung fettiger und/oder sebumglänzender Kopfhaut (Skalp) gemäß einem der Patentansprüche 1-17 zur Verbesserung des optischen Erscheinungsbildes der Kopfhaut/des Skalps.

Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin mindestens einen konditionierenden Wirkstoff enthalten. Unter konditionierenden Wirkstoffen sind erfindungsgemäß solche Substanzen zu verstehen, die auf keratinische Materialien, insbesondere auf die Kopfhaut, aufziehen und die physikalischen und sensorischen Eigenschaften sowohl der Kopfhaut als auch des Produktes als solchem verbessern. Konditionierungsmittel glätten die oberste Schicht der Kopfhaut und machen sie weich und geschmeidig. Über die Auswahl der Konditionierungsmittel (fettig - weniger fettig, schnell oder langsam spreitend, schnell oder langsam in die Kopfhaut einziehend und so weiter) lässt sich das Hautgefühl des gesamten Produktes einstellen. Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-aikylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglycoldioleat oder Propylenglycoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, EiLecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.
Erfindungsgemäß besonders bevorzugt verwendete Fettstoffe sind ausgewählt aus Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethicone, Dimethiconol, Amodimethicone oder Trimethylsilylamodimethicone, wobei Decamethylcyclopentasiloxan, Dodecamethylcyclohexasiloxan, Dimethicone, Dimethicone Copolyole und Dimethiconol sowie deren Mischungen besonders bevorzugt sind. Es wurde für den Fachmann überraschend festgestellt, dass die Siliconverbindungen, insbesondere Decamethylcyclopentasiloxan, Dodecamethylcyclohexasiloxan, Dimethicone, Dimethicone Copolyole und Dimethiconol sowie deren Mischungen, die mattierende Wirkung des partikelförmigen, sebumsorbierenden Wirkstoffs weiter steigern können.
Erfindungsgemäß besonders bevorzugt verwendete kosmetische oder dermatologische Zusammensetzungen zur mattierenden Behandlung der Kopfhaut, die in einem geeigneten kosmetischen oder dermatologischen Träger mindestens einen partikelförmigen sebumsorbierenden Wirkstoff enthalten, sind dadurch gekennzeichnet, dass sie weiterhin mindestens einen konditionierenden Wirkstoff, ausgewählt aus Decamethylcyclopentasiloxan, Dodecamethylcyclohexasiloxan, Dimethicone, Dimethicone Copolyole und Dimethiconol sowie deren Mischungen, enthalten.
Die Einsatzmenge der Fettstoffe beträgt 0,1 - 30 Gew.%, bevorzugt 0,5 - 10 Gew.%, besonders bevorzugt 1 - 5 Gew.%, und außerordentlich bevorzugt 2 - 4 Gew.%, jeweils bezogen auf das gesamte Kopfhautbehandlungsmittel.

Bevorzugt liegen die erfindungsgemäß verwendeten kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines alkoholischen oder glycolischen Gels, einer ein- oder mehrphasigen Lösung, eines Schaumes oder eines Puders vor.

Die Mittel können auch in wasserfreier Form, wie beispielsweise einem Öl oder einem Lipogel, dargereicht werden. Hierbei kann der Träger ein pflanzliches oder tierisches Öl, ein Mineralöl, ein synthetisches Öl oder eine Mischung solcher Öle sein, wobei Siliconverbindungen als Öle bevorzugt sind.
In einer besonders bevorzugten Ausführungsform der Erfindung liegen die erfindungsgemäß verwendeten Zusammensetzungen als wässriges oder ethanolisches oder glycolisches Gel vor.
Erfindungsgemäß wird unter einem wässrigen Gel eine Zusammensetzung verstanden, die mindestens 80 Gew.-% Wasser, mindestens eine gelbildende Substanz und maximal 5 Gew.-% Fettstoffe enthält.
Erfindungsgemäß wird unter einem ethanolischen Gel eine Zusammensetzung verstanden, die mindestens 30 Gew.-% Ethanol, mindestens eine gelbildende Substanz und maximal 5 Gew.-% Fettstoffe enthält.

Erfindungsgemäß wird unter einem glycolischen Gel eine Zusammensetzung verstanden, die mindestens 20 Gew.-% eines mehrwertigen C₂-C₉-Alkohols, mindestens eine gelbildende Substanz und maximal 5 Gew.-% Fettstoffe enthält.
In einer besonders bevorzugten Ausführungsform der Erfindung liegen die erfindungsgemäß verwendeten Zusammensetzungen als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.
In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-düsostearat (Handelsprodukt Lameform^{®} TGI) sowie lineare und verzweigte C₈-C₃₀-Fettsäuren und deren Na-, K-, Ammonium-, Ca-, Mg- und Zn - Salze.
Die erfindungsgemäß verwendeten Zusammensetzungen enthalten die Emulgatoren bevorzugt in Mengen von 0,1 bis 25 Gew.-%, besonders bevorzugt 0,5 - 15 Gew.-%, bezogen auf die gesamte Zusammensetzung.
In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt eine Behenylgruppe, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, ist. Besonders bevorzugt ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat. Die Anwesenheit von Emulgatoren der allgemeinen Formel R¹ - O - R², insbesondere von Behenylalkohol, kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.
Weitere erfindungsgemäß bevorzugt verwendete Zusatzstoffe sind Verdickungsmittel, insbesondere für die bevorzugten Darreichungsformen als wässriges, ethanolisches oder glycolisches Gel. Besonders bevorzugt verwendete Verdickungsmittel sind natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS, Simulgel^{®} EPG und Simulgel^{®} EG der Firma SEPPIC sowie Aristoflex^{®} AVC von Clariant. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀-₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).
Geeignete nichtionische Polymere sind beispielsweise Polyvinylalkohole, die teilverseift sein können, z. B. die Handelsprodukte Mowiol^{®} sowie VinylpyrrolidonNinylester-Copolymere und Polyvinylpyrrolidone, die z. B. unter dem Warenzeichen Luviskol^{®} (BASF) vertrieben werden, weiterhin Cellulosederivate, insbesondere Hydroxypropylmethylcellulose, Hydroxypropylcellulose und Hydroxyethylcellulose.

In einer weiteren besonders bevorzugten Ausführungsform der Erfindung enthalten die erfindungsgemäß verwendeten Zusammensetzungen mindestens ein Tensid.
Unter dem Begriff Tenside werden oberflächenaktive Substanzen verstanden, die eine anionische oder kationische Ladung im Molekül tragen. Ebenfalls kann im Molekül sowohl eine anionische als auch kationische Ladung vorhanden sein. Diese zwitterionischen oder amphoteren oberflächenaktiven Substanzen können erfindungsgemäß ebenfalls eingesetzt werden. Weiterhin können die oberflächenaktiven Substanzen auch nicht-ionisch sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglycolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglycolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (VI), 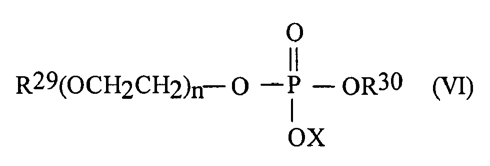 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglycolester der Formel R³⁵CO(AlkO)ₙSO₃M, in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (VIII), 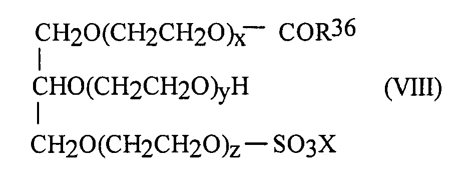
in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VIII) eingesetzt, in der R³⁶CO für einen linearen Acylrest mit 8 bis 18 Kohlenstoffatomen steht. Monoglyceridsulfate und Monoglyceridethersulfate werden beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben.

Bevorzugte anionische Tenside sind Alkylsulfate, Alkylpolyglycolethersulfate und Ethercarbonsäuren mit 10 bis 18 C-Atomen in der Alkylgruppe und bis zu 12 Glycolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxylierte Triglyceride,
- alkoxylierte Fettsäurealkylester der Formel R³⁷CO-(OCH₂CHR³⁸)_{w}OR³⁹, (IX),
   in der R³⁷ CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungsprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolyglycoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglycoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglyckoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Auch die alkoxylierten Homologen der genannten Alkylpolyglycoside können erfindungsgemäß eingesetzt werden. Diese Homologen können durchschnittlich bis zu 10 Ethylenoxid- und/oder Propylenoxideinheiten pro Alkylglycosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Bevorzugt wird mindestens ein Tensid in einer Gesamtmenge von 0,1 - 30 Gew.%, bevorzugt 0,5 - 15 Gew.%, besonders bevorzugt von 1 - 10 Gew.%, jeweils bezogen auf die gesamte erfindungsgemäß verwendete Zusammensetzung, eingesetzt.

In einer bevorzugten Ausführungsform können nicht-ionische, zwitterionische und/oder amphotere Tenside sowie deren Mischungen bevorzugt sein.

Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid (INCI-Bezeichnung Dipalmitoylethyl Dimonium Chloride), sowie Distearoylethyl Hydroxyethylmonium Methosulfate, das beispielsweise in einer Mischung mit Cetearylalkohol unter der Handelsbezeichnung Dehyquart^{®} F-75 von Cognis erhältlich ist, Dipalmitoylethyl Hydroxyethylmonium Methosulfate, das beispielsweise in einer Mischung mit Cetearylalkohol und Ceteareth-20 unter der Handelsbezeichnung Dehyquart^{®} C-4046 von Cognis erhältlich ist, Dicocoylethyl Hydroxyethylmonium Methosulfate, das beispielsweise in einer Propylenglycollösung unter der Handelsbezeichnung Dehyquart^{®} L 80 von Cognis erhältlich ist, und N-Methyl-N-(2-hydroxyethyl)-N,N-bis-(acyloxyethyl)-ammonium methosulfate, das beispielsweise in einer Isopropanollösung unter der Handelsbezeichnung Dehyquart^{®} AU-35 von Cognis erhältlich ist, sind Beispiele für erfindungsgemäß besonders bevorzugt verwendete Esterquats. Die Esterquats sind erfindungsgemäß besonders bevorzugt verwendete kationische Tenside aufgrund ihrer hohen Hautmilde und Hautverträglichkeit.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung des erfindungsgemäß verwendeten Wirkstoffes durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäß verwendeten Mittel enthalten die Emulgatoren bevorzugt in einer Gesamtmenge von 0,1 - 25 Gew.-%, besonders bevorzugt 0,5 - 10 - 15 Gew.-%, außerordentlich bevorzugt 1 - 5 Gew.-%, jeweils bezogen auf das gesamte Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997)Seite 1764, aufgeführten Definitionen enthalten. Nichtionogene Emulgatoren mit einem HLB-Wert von 10-15 können erfindungsgemäß besonders bevorzugt sein.

Unter den genannten Emulgatoren-Typen können die Emulgatoren, die kein Ethylenoxid und/oder Propylenoxid im Molekül enthalten, ganz besonders bevorzugt sein.

Weitere bevorzugte Zusatzstoffe sind Konservierungsmittel, Lösungsmittel wie Ethanol, Isopropanol, Ethylenglycol, Propylenglycol, Propylenglycolmonoethylether, Glycerin und Diethylenglycol, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.

### Wässriges Gel zur mattierenden Kopfhautbehandlung

| | |
|---|---|
| Micropearl 310 | 3,00 |
| Ethanol, 96 %, vergällt | 5,00 |
| Glycerin, 86 % | 0,50 |
| Sepigel 305 | 1,70 |
| D-Panthenol | 0,10 |
| Phenoxyethanol | 0,20 |
| Allantoin | 0,10 |
| d,l-alpha-Bisabolol | 0,20 |
| Methylparaben | 0,10 |
| Dow Corning 1403 Fluid | 2,00 |
| Parfum | 0,30 |
| Wasser | ad 100 |

Das Gel wurde auf die kahlen Stellen der Kopfhaut/des Skalps der Probanden aufgetragen. Es wurde eine deutliche Mattierung der zuvor auffällig glänzenden Kopfhaut erzielt. Die mattierende Wirkung hielt mehrere Stunden an.

### 1. Oel-in-Wasser-Emulsionen

### 1. Mattierende Cremes

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dry Flo Plus | 1,00 | 1,00 | 1,00 | 1,00 |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Phenoxyethanol | 0,90 | 0,50 | 0,90 | 0,90 |
| Herbasol Destillat Salbei | 1,00 | - | - | - |
| Herbasol Destillat Hamamelis | - | 1,00 | 2,00 | - |
| D-Panthenol | 0,70 | 0,525 | 0,60 | - |
| Epica | 0,05 | 0,05 | - | - |
| DSH-CN | 5,00 | 5,00 | 3,00 | 3,00 |
| Herbasol Destillat Weißer Tee | - | - | 0,50 | 0,50 |
| Hydroglycolic Extract of Ginseng | - | 0,45 | - | 0,45 |
| Seboregul 2 | - | - | 2,00 | - |
| Asebiol LS 2539 BT 2 | - | - | - | 0,50 |
| Micropearl M 310 | 2,00 | 1,00 | 3,00 | 1,50 |
| Perfume Aqua | 0,10 ad 100 | 0,10 ad 100 | 0,10 ad 100 | 0,10 ad 100 |

### 2. Mattierende Cremes:

| | 1 | 2 | 3 | 4 | 5 |
|---|---|---|---|---|---|
| Lipoid S 75-3 | 0,50 | 0,50 | 0,50 | 0,50 | 0,50 |
| Tocopherylacetat | 0,50 | 0,50 | 0,50 | 0,50 | 0,50 |
| Cutina MD | 1,00 | 1,00 | 1,00 | 1,00 | 1,00 |
| Lanette 22 | 2,00 | 2,00 | 2,00 | 2,00 | 2,00 |
| Baysilone-Öl M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer 140 | 0,30 | 0,30 | 0,30 | 0,30 | 0,30 |
| DSH-C N | 1,00 | - | - | - | - |
| Propylenglycol | 5,00 | 5,00 | 5,00 | 5,00 | 5,00 |
| Micropearl M 310 | 2,00 | 1,00 | 3,00 | 1,50 | 2,00 |
| Natrulon RC50DG | - | - | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

### 3. Mattierende Cremes

| | 1 | 2 | 3 |
|---|---|---|---|
| Sisterna SP 30 C | 2,00 | 2,00 | 2,00 |
| Sisterna SP 70 C | 0,8 | 0,80 | 0,80 |
| Propylparaben | 0,20 | 0,20 | 0,20 |
| Methylparaben | 0,20 | 0,20 | 0,20 |
| Ethanol | 5,00 | 3,00 | - |
| Dry Flo Plus | 1,00 | 1,00 | 1,00 |
| Micropearl M 310 | 2,00 | 1,00 | 3,00 |
| Perfume | 0,30 | 0,30 | 0,30 |
| Cetearyl Alcohol | 1,00 | 1,00 | 1,00 |
| Fucogel 1000 | 1,00 | 1,00 | 1,00 |
| Herbasol Destillat Salbei | 1,00 | - | - |
| Herbasol Destillat Hamamelis | - | 1,00 | 2,00 |
| D-Panthenol | 0,70 | 0,525 | 0,60 |
| Keltrol SF | 0,20 | - | 0,20 |
| Aristoflex AVC | - | 0,50 | - |
| 1,6-Hexandiol | 5,00 | - | - |
| Glycerin 86 % | 10,00 | 5,00 | 10,00 |
| Dow Corning 200 Fluid 0,65 cSt | 7,00 | - | - |
| Dow Corning 245 | - | 5,00 | 5,00 |
| Cetiol 868 | 2,00 | - | 2,00 |
| Aqua | ad 100 | ad 100 | ad 100 |

### 4. Mattierende Cremes:

| | 1 | 2 | 3 |
|---|---|---|---|
| Montanov 202 | 5,00 | 5,00 | 5,00 |
| Cutina MD | 0,50 | 0,50 | 0,50 |
| Cetearyl Alcohol | 1,00 | 1,00 | 1,00 |
| Dibutyl Adipate | 9,00 | 9,00 | 9,00 |
| Controx KS | 0,05 | 0,05 | 0,05 |
| Dow Corning 245 | - | 5,00 | 5,00 |
| Dow Corning 9040 | 1,0 | 1,0 | - |
| Micropearl M 310 | 2,00 | 3,00 | 1,00 |
| Perfume | 0,30 | 0,30 | 0,30 |
| Cetearyl Alcohol | 1,00 | 1,00 | 1,00 |
| D-Panthenol | - | 0,525 | 0,60 |
| DSH-CN | 5,00 | 5,00 | 3,00 |
| Hydroglycolic Extract of Ginseng | - | 0,45 | - |
| Keltrol SF | - | - | 0,20 |
| Aristoflex AVC | - | 0,50 | - |
| 1,6-Hexandiol | 6,00 | 6,00 | - |
| Glycerin 86 % | 3,00 | 5,00 | 10,00 |
| Propylenglycol | 2,00 | 2,00 | 2,00 |
| Dow Corning 200 Fluid 0,65 cSt | 7,00 | 7,00 | 7,00 |
| Tego Carbomer 140 | 0,30 | 0,30 | 0,30 |
| Phenonip | 0,50 | 0,50 | 0,50 |
| Aqua | ad 100 | ad 100 | ad 100 |

Die vorstehend aufgeführten Cremes wurden auf die kahlen Stellen der Kopfhaut/des Skalps der Probanden aufgetragen. Es wurde eine deutliche Mattierung der zuvor auffällig glänzenden Kopfhaut erzielt. Die mattierende Wirkung hielt mehrere Stunden an.

### 2.1 Mattierende Kopfhaut-Tonics:

| | 1 | 2 | 3 |
|---|---|---|---|
| Dipropylenglycol | 10,00 | 10,00 | 10,00 |
| Chlorhexidindigluconate | 1,00 | 1,00 | 1,00 |
| Poloxamer-184 | 3,00 | 3,00 | 3,00 |
| Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Chlorella Vulgaris Extract | 0,50 | 0,50 | 0,50 |
| Seboregul 2 | - | - | 2,00 |
| Micropearl M 310 | 2,00 | 3,00 | 1,00 |
| Perfume | 0,20 | 0,20 | 0,20 |
| Aqua | ad 100 | ad 100 | ad 100 |

### 2.2 Reinigungsgele:

| | 1 | 2 | 3 |
|---|---|---|---|
| Carbomer | 1,40 | 1,40 | 1,40 |
| Sorbitol | 2,10 | 2,10 | 2,10 |
| Sodium Benzoate | 0,40 | 0,40 | 0,40 |
| Plantaren^{®} 1200 | 7,50 | 7,50 | 7,50 |
| Dehyton^{®} K | 3,40 | 3,40 | 3,40 |
| Disodium Laureth Sulfosuccinate | 5,00 | 5,00 | 5,00 |
| Cetiol^{®} HE | 0,50 | 0,50 | 0,50 |
| Lamesoft^{®} PO 65 | 5,00 | 5,00 | 5,00 |
| Controx KS | 0,05 | 0,05 | 0,05 |
| Sodium PCA | 1,60 | 1,60 | - |
| Pantolactone | 1,00 | 1,00 | - |
| Tetrasodium EDTA | 0,25 | 0,25 | 0,25 |
| Sodium Lactate | 1,80 | - | - |
| Micropearl M 310 | 3,00 | 2,00 | 4,00 |
| Panthenol | 0,50 | 0,50 | 0,50 |
| Caomint | 1,00 | - | 2,00 |
| Calmosensine | 1,00 | 2,00 | - |
| Acnacidol PG | 0,10 | 0,50 | - |
| Perfume | 0,40 | 0,40 | 0,40 |
| Aqua | ad 100 | ad 100 | ad 100 |

Die vorstehend aufgeführten Reinigungsgele wurden auf die Kopfhaut/den Skalp der Probanden aufgetragen, einmassiert und anschließend mit Wasser abgespült oder mit einem Pad oder Tuch abgewischt. Es wurde eine deutliche Mattierung der zuvor auffällig glänzenden Kopfhaut erzielt. Die mattierende Wirkung hielt mehrere Stunden an.

### Liste der verwendeten Rohstoffe

| Handelsname | INCI-Bezeichnung | Lieferant/Hersteller |
|---|---|---|
| | | |
| Acnacidol PG | Sebacic acid, 10-hydroxydecanoic acid, 1,10-decanediol | Vincience |
| Ajidew^{®} NL 50 | Sodium PCA | AJINOMOTO |
| Asebiol^{®} LS 2539 BT 2 | Aqua, Hydrolyzed Yeast Protein, Pyridoxine, Niacinamide, Glycerin, Panthenol, Allantoin, Biotin | Laboratoires Sero-biologiques |
| Baysilone-Öl M 350 | Dimethicone | GE Bayer |
| | | Silicones |
| Biodocarb^{®} | lodopropynyl Butylcarbamate | MILKER & GRÜNING |
| Biopeptide CL | N-Palmitoyl-Gly-His-Lys | Sederma |
| Biopeptide EL | N-Palmitoyl-Val-Gly-Val-Ala-Pro-Gly | Sederma |
| BiSCos Glynarin PF | AQUA (WATER), ALCOHOL, PHENOXY-ETHANOL, AMMONIUM GLYCYRRHIZATE, TANNIC ACID, NARINGINE | Biesterfeld |
| Brij 30 | Laureth-4 | Uniqema |
| Calmosensine | BUTYLENE GLYCOL, WATER, LAURETH-3, HYDROXYETHYLCELLULOSE, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 Cetiol HE | Acrylates/C10-30 Alkyl Acrylate Crosspolymer | Noveon |
| | PEG-7 Glyceryl Cocoate | Cognis |
| Cetiol^{®} PGL | Hexyldecanol, Hexyldecyl Laurate | Cognis |
| Controx KS | Tocopherol, Hydrogenated Palm Glycerides Citrate | Cognis |
| Cosmedia Guar^{®} C 261 | Guar Hydroxypropyl Trimonium Chloride | Cognis |
| Crodarom Chardonnay L | Propylene Glycol, Water, Vitis Vinifera (Grape) Seed Extract | Croda |
| Cutina CBS | Glyceryl Stearate / Cetearyl Alcohol / Cetyl Palmitate / Cocoglycerides | Cognis |
| Cutina GMS | Glyceryl Stearate (Mischung aus Glyceryl-mono- und distearat) | Cognis |
| Cutina MD | Glyceryl Stearate | Cognis |
| DC^{®} 190 | Dimethicone | Dow Corning |
| Dehyton K | AQUA (WATER), COCAMIDOPROPYL BETAINE (29 - 32 Gew.-%) | Cognis |
| Deliner | Zea Mays (Corn) Kernel Extract, Butylene Glycol, Xanthan Gum | Coletica |
| DERMOSOFT OCTIOL | 1,2-Octandiol | Dr. Straetmans |
| Dow Corning 1403 Fluid | Cyclomethicone (86 - 88 Gew.-%), Dimethiconol (12-14 Gew.-%) | Dow Corning |
| Dow Corning 1501 Fluid | Cyclomethicone (85 - 86 Gew.-%), Dimethiconol (14-15 Gew.-%) | Dow Corning |
| Dow Corning 200 (0,65 cSt) | Dimethicone | Dow Corning |
| Dow Corning 9040 | Cyclomethicone/ Dimethicone Crosspolymer | Dow Corning |
| Dow Corning^{®}200 Fluid, 5 cSt. | Dimethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline H | PEG-40 Hydrogenated Castor Oil, PPG-2-Ceteareth-9, Pyrus Malus (Apple) Fruit Extract | Seporga |
| Ederline L | Hexyldecanol, Pyrus Malus (Apple) Fruit Extract | Seporga |
| EMULGADE^{®} SE | Glyceryl Stearate, Ceteareth-20, Cetea-reth-12, Cetearyl Alcohol, Cetyl Palmitate | Cognis |
| Epica | AQUA (WATER), ALCOHOL, PINUS SYLVESTRIS BARK EXTRACT, RIBES NIGRUM (BLACK CURRANT) LEAF EXTRACT | Greentech (Rahn) |
| Eumulgin B 2 | Ceteareth-20 | Cognis |
| Eumulgin B 3 | Ceteareth-30 | Cognis |
| Eumulgin 05 | Oleth-5 | Cognis |
| Eusolex^{®} OCR | Octocrylene | Merck KGaA |
| Exsy Algine | ACETYL CITRULL AMIDO ARGININE | Exsymol |
| Fucogel 1000 | Biosaccharide Gum-1 | Solabia |
| Germall^{®} 115 | Imidazolidinyl Urea | Sutton Laboratories |
| Glistin | L-Glutamylaminoethyl indole | Exsymol |
| Glucamate^{®}DOE 120 | PEG-120 Methyl Glucose Dioleate | AMERCHOL |
| Hibiscin^{®} HP-LS-9198 | Water, Hibiscus esculentus Seed Extract, Phenoxyethanol | Laboratoires Särobiologiques |
| Honeyquat^{®} 50 | Hydroxypropyltrimonium Honey | BROOKS |
| Hydagen CMF | Chitosan Glycolate | Cognis |
| Hydrovance | (2-Hydroxyethyl)harnstoff | National Starch |
| Jaguar HP 105 | HYDROXYPROPYL GUAR | Rhodia |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} O | Cetearyl Alcohol | Cognis |
| LANETTE^{®} 22 | Behenyl Alcohol | Cognis |
| Lipobelle Soyaglycone | Alcohol, Polysorbate 80, Soy Isoflavones | Mibelle AG Cosmetics |
| Lipoid S 75-3 | Aqua, Lecithine | Lipoid GmbH |
| Lotus Germ Extract | Water, Butylene Glycol, Nelumbo Nucifera Germ Extract | Maruzen |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Micropearl M 310 | Methyl Methacrylate Crosspolymer | Seppic |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| MYRITOL^{®} 312 | Caprylic/Capric Triglyceride | Cognis |
| MYRITOL^{®} PC | PROPYLENE GLYCOL DICAPRYLATE/DICAPRATE | Cognis |
| Natrulon RC 50 DG | WATER, CARNITINE, POLYGLYCERIN-10 | Lonza |
| Neutrol TE | TETRAHYDROXYPROPYL ETHYLENEDIAMINE | BASF |
| Novata AB | Cocoglycerides | Cognis |
| Olea europ. Fol extr. S. sicc. | Olea Europea (Olive) Leaf Extract | Fruitarom |
| Omega-CH-Aktivator | Tripeptide-1 | GfN |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Phenonip^{®} | Phenoxyethanol, Methylparaben, Ethylparaben, Propylparaben, Butylparaben, ca. 28 % Aktivsubstanz | NIPA |
| Photosomes | Aqua, Lecithine, Plancton Extract | AGI Dermatics |
| Phytodermin | SOYBEAN PROTEIN GLYCINE SOJA (Linne) | CLR Chem. Laboratorium Kurt Richter |
| Phytokine | HYDROLYZED SOY PROTEIN | Coletica |
| Plantacare^{®} 818 UP | Coco Glucoside, ca. 50 % Aktivsubstanz | Cognis |
| Plantaren^{®} 1200 | Lauryl Glucoside, ca. 50 % Aktivsubstanz | Cognis |
| Plantaren^{®} 2000 | Decyl Glucoside, ca. 50 % Aktivsubstanz | Cognis |
| Polymer JR 400^{®} | Polyquaternium-10 | UNION CARBIDE |
| Ridulisse C | HYDROLYZED SOY PROTEIN | Silab |
| Sambucus AO | Water Glycerin, Sambucus Nigra Flower Extract | Alpaflor/Center-chem |
| Seboregul 2 | BUTYLENE GLYCOL, AQUA (WATER), SPIRAEA ULMARIA EXTRACT | Silab |
| Sensiva^{®} SC 50 | 2-Ethylhexylglycerinether | Schülke & Mayr |
| Sepigel^{®}305 | Polyacrylamide, C₁₃-C₁₄ Isoparaffin, Laureth-7 | SEPPIC |
| Sepivinol R | Wine Extract (Polyphenolreicher Extrakt aus Rotwein) | Seppic |
| Sepilift DPHP | Dipalmitoyl Hydroxyproline | Seppic |
| Silymarin Phytosome | Silybum Marianum Extract and Phospholipids | Indena SpA |
| Simulgel NS | Hydroxyethyl Acrylate / Sodium Acryloyl-dimethyl Taurate Copolymer / Squalane / Polysorbate 60 | Seppic |
| Soy Protein Isolate | | Protein Technology International |
| Stenol 16/18 | Cetearylalkohol | Cognis |
| Surfadone LP 300 | N-Laurylpyrrolidon | ISP |
| Symdiol 68 | 1,2-Octandiol, 1,2-Hexandiol | Symrise |
| SYN^{®}-COLL | Water, Palmitoyl-Lys-Val-Lys | Pentapharm |
| Texapon^{®} NSO | Sodium Laureth Sulfate, Aqua (ca. 28 % Aktivsubstanz) | Cognis |
| Texapon^{®} SB 3 | Aqua, Disodium Laureth Sulfosuccinate, (ca. 40 % Aktivsubstanz), Citric Acid | Cognis |
| Tioveil^{®}-AQ-N | Cl 77891 (Titanium Dioxide),Alumina, Silica, Sodium Polyacrylate | Uniqema (Tioxide Specialties) |
| Tween^{®}-80 | Polysorbate-80 | |
| Ultrahold 8 | ACRYLATES/t-BUTYLACRYLAMIDE COPOLYMER | BASF |
| Ultrasomes | Aqua, Lecithine, Micrococcus Luteus Extract | AGI Dermatics |
| Uvinul MS 40 | BENZOPHENONE-4 | BASF |
| Vegetal Filling Spheres | CAPRYLIC/CAPRIC TRIGLYCERIDE, SILICA DIMETHYL SILYLATE, HYDROLYZED WHEAT PROTEIN, BUTYLENE GLYCOL, PHENOXYETHANOL, METHYLPARABEN, ETHYLPARABEN, PROPYLPARABEN, BUTYLPARABEN, ISOBUTYLPARABEN | Coletica |

## Patentansprüche

1. Verwendung einer kosmetischen oder dermatologischen Zusammensetzung, enthaltend in einem geeigneten kosmetischen oder dermatologischen Träger mindestens einen partikelförmigen, sebumsorbierenden Wirkstoff, zur nicht-therapeutischen, kosmetischen topischen mattierenden Behandlung der Kopfhaut (Skalp) und/oder zur nicht-therapeutischen, kosmetischen topischen Behandlung fettiger und/oder sebumglänzender Kopfhaut (Skalp).

2. Verwendung gemäß Anspruch 1, **dadurch gekennzeichnet, dass** der partikelförmige, sebumsorbierende Wirkstoff ausgewählt ist aus anorganischen und organischen kosmetischen Adsorbentien mit mittleren Partikeldurchmessern von 0,1 - 100 µm, bevorzugt 0,5 - 50 µm, besonders bevorzugt 5 - 30 µm und außerordentlich bevorzugt 10 - 25 µm.

3. Verwendung gemäß Anspruch 1 oder 2, **dadurch gekennzeichnet, dass** der partikelförmige, sebumsorbierende Wirkstoff ausgewählt ist aus folgenden anorganischen Adsorbentien: Kieselsäuren, insbesondere Aerosil^{®}-Typen, Kieselgelen, Siliciumdioxid, Tonen und Schichtsilicaten, insbesondere Bentonite oder Kaolin, Magnesiumaluminiumsilikaten, insbesondere Talkum, und Bornitrid, sowie Mischungen der genannten Substanzen.

4. Verwendung gemäß einem der Ansprüche 1 - 3, **dadurch gekennzeichnet, dass** der partikelförmige, sebumsorbierende Wirkstoff ausgewählt ist aus folgenden organischen Adsorbentien: Stärken und Stärkederivaten, die chemisch und/oder physikalisch modifiziert sein können, Cellulosepulvern, Lactoglobulinderivaten, insbesondere Natrium-C₈₋₁₆-Isoalkylsuccinyllactoglobulinsulfonat, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, sowie Mischungen hiervon.

5. Verwendung gemäß einem der Ansprüche 1 - 4, **dadurch gekennzeichnet, dass** der partikelförmige, sebumsorbierende Wirkstoff ausgewählt ist aus vernetzten Polymerpulvern.

6. Verwendung gemäß einem der Ansprüche 1 - 5, **dadurch gekennzeichnet, dass** der partikelförmige, sebumsorbierende Wirkstoff ausgewählt ist aus vernetzten Polymethacrylaten und vernetzten Polymethylmethacrylaten.

7. Verwendung gemäß einem der Ansprüche 1 - 6, **dadurch gekennzeichnet, dass** der partikelförmige, sebumsorbierende Wirkstoff Hohlräume aufweist.

8. Verwendung gemäß einem der Ansprüche 1 - 7, **dadurch gekennzeichnet, dass** der partikelförmige, sebumsorbierende Wirkstoff halbkugelförmige Partikel aufweist.

9. Verwendung gemäß Anspruch 8, **dadurch gekennzeichnet, dass** mindestens 50 Gew.-%, bevorzugt mindestens 80 Gew.-%, der Partikel halbkugelförmig sind.

10. Verwendung gemäß Anspruch 8 oder 9, **dadurch gekennzeichnet, dass** der partikelförmige, sebumsorbierende Wirkstoff halbkugelförmige Partikel mit Hohlräumen aufweist.

11. Verwendung gemäß Anspruch 10, **dadurch gekennzeichnet, dass** mindestens 50 Gew.-%, bevorzugt mindestens 80 Gew.-% der Partikel halbkugelförmig mit Hohlräumen sind.

12. Verwendung gemäß einem der Ansprüche 1-11, **dadurch gekennzeichnet, dass** die kosmetische oder dermatologische Zusammensetzung mindestens einen partikelförmigen sebumsorbierenden Wirkstoff in einer Gesamtmenge von 0,1 bis 10 Gew.-%, bevorzugt 0,5 bis 1 bis 7 Gew.-%, besonders bevorzugt 2 bis 5 Gew.-%, und außerordentlich bevorzugt 3 bis 4 Gew.-%, jeweils bezogen auf die gesamte Zusammensetzung, enthält.

13. Verwendung gemäß einem der Ansprüche 1 - 12, **dadurch gekennzeichnet, dass** die kosmetische oder dermatologische Zusammensetzung mindestens einen sebumregulierenden Wirkstoff enthält.

14. Verwendung gemäß Anspruch 13, **dadurch gekennzeichnet, dass** der sebumregulierende Wirkstoff ausgewählt ist aus Azelainsäure, Azelainsäurederivaten, insbesondere dem Azelainsäurederivat Potassium Azeloyl Diglycinate, Sebacinsäure, 10-Hydroxydecansäure, 1,10-Decandiol, Mischungen aus Sebacinsäure, 10-Hydroxydecansäure und 1,10-Decandiol, Glycyrrhizin, sowie deren Salzen, Gerbsäure (tannic acid) und deren Salzen, Gallotanninen, Naringin, Mischungen aus Glycyrrhizin(salzen), Gerbsäure(salzen) und/oder Gallotanninen und Naringin, Extrakten aus Spiraea Ulmaria, wasser- und öllöslichen Extrakten aus Hamamelis, Klettenwurzel oder Brennessel, Zimtbaumextrakt, Chrysanthemenextrakt, Hefeproteinhydrolysaten, mindestens einer Vitamin B6-Komponente, die ausgewählt ist aus Pyridoxin, Pyridoxinhydrochlorid, Pyridoxin-5'-monophosphat, Pyridoxal, Pyridoxal-5'-monophosphat, Pyridoxamin, Pyridoxaminhydrochlorid und Pyridoxamin-5'-monophosphat sowie Mischungen dieser Komponenten, Biotin und Biotinestern sowie PEG-8 Isolauryl Thioether.

15. Verwendung gemäß Anspruch 13 oder 14, **dadurch gekennzeichnet, dass** der sebumregulierende Wirkstoff nicht als 5-alpha-Reductase-Inhibitor wirkt.

16. Verwendung gemäß einem der vorstehenden Ansprüche, **dadurch gekennzeichnet, dass** die kosmetische oder dermatologische Zusammensetzung weiterhin mindestens einen kosmetischen Wirkstoff enthält, der ausgewählt ist aus
- Vitaminen, Provitaminen und Vitaminvorstufen der Gruppen A, B, C, E, H und K und den Estern und Salzen der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- hautberuhigenden und
- feuchtigkeitsspendenden Wirkstoffen sowie
- Mischungen dieser Wirkstoffe.

17. Verwendung gemäß einem der vorstehenden Ansprüche, **dadurch gekennzeichnet, dass** die kosmetische oder dermatologische Zusammensetzung ein wässriges oder ethanolisches oder glycolisches Gel darstellt.

18. Verwendung einer kosmetischen oder dermatologischen Zusammensetzung gemäß einem der Ansprüche 1 - 17 zur Verbesserung des optischen Erscheinungsbildes der Kopfhaut (Skalp).
